# EUROPEAN PATENT APPLICATION

(11) **EP 2 946 808 A1**
(43) Date of publication of application: **25.11.2015**
(21) Application number: 14756483.5
(22) Date of filing: 28.02.2014
(51) Int. Cl.: A61N 5/10, A61B 6/00, G01T 1/29, G21K 1/02

(54) **THERAPY PLANNING DEVICE, SYSTEM FOR PLANNED THERAPY, METHOD FOR MAKING THERAPY PLAN, AND PROGRAM**

(30) Priority: 28.02.2013 JP 2013039754
(71) Applicant: Mitsubishi Heavy Industries, Ltd., Tokyo 108-8215 (JP); Kyoto University, Kyoto-shi, Kyoto 606-8501 (JP)
(72) Inventor: MURAKI, Satoshi, Tokyo 108-8215 (JP); NIINO, Tadashi, Tokyo 108-8215 (JP); MIYABE, Yuki, Kyoto-shi Kyoto 606-8501 (JP); KANEKO, Shuji, Kyoto-shi Kyoto 606-8501 (JP); MIZOWAKI, Takashi, Kyoto-shi Kyoto 606-8501 (JP); HIRAOKA, Masahiro, Kyoto-shi Kyoto 606-8501 (JP)
(74) Representative: Hoffmann Eitle
(86) International application number: PCT/JP2014/055090
(87) International publication number: WO 2014/133139

(57) **Abstract**

A therapy planning device includes: a condition acquiring unit configured to acquire therapy conditions including a position of an affected area, a necessary dose for the affected area, and a threshold dose for an area other than the affected area; a first planning unit configured to plan a first irradiation of irradiating a first irradiation field including the entire affected area to radiation; and a second planning unit configured to make a therapy plan satisfying the therapy conditions in combination of the first irradiation planned by the first planning unit and irradiation of an irradiation field including only part of the affected area.

## Description

### [Technical Field]

The present invention relates to a therapy planning device, a planned therapy system, a therapy plan making method, and a program.

Priority is claimed on Japanese Patent Application No. 2013-039754, filed February 28, 2013, the content of which is incorporated herein by reference.

### [Background Art]

Radiation therapy is known as a form of cancer therapy. In radiation therapy, therapeutic radiation (such as an electron beam, X-rays, a proton beam, a heavy particle beam, γ-rays, and a neutron beam) is concentrated on an affected area, and therapy is executed by irradiating only cancer cells with a lethal dose of radiation while suppressing side effects of radiation on normal cells.

Here, an affected area to be irradiated is generally in part that is not visible from the outside. Accordingly, in a state in which a patient is seated on a couch, an internal image such as an X-ray image, a magnetic resonance imaging (MRI) image, or a positron emission tomography (PET) image is acquired and a position is determined in order to irradiate an affected area with therapeutic radiation. A human body moves due to respiration, muscle relaxation, or the like, and movement of a lung field and the vicinity thereof due to respiration is especially large.

There is a possibility of an affected area moving during irradiation with therapeutic radiation due to the movement of the body. Accordingly, it may be confirmed (monitored) in real time that an affected area is accurately irradiated with therapeutic radiation by receiving therapeutic radiation passing through the body using an electric portal imaging device (EPID) or the like to acquire a radiographic image.

On the other hand, intensity modulated radiation therapy (IMRT) is known as therapy in which therapeutic radiation is concentrated on an affected area (see Patent Literatures 1 and 2). In intensity modulated radiation therapy, which is presently widely used in clinical sites, a plurality of therapeutic radiation beams having irradiation fields smaller than an affected area are superimposed using a multi-leaf collimator (MLC), a fine linear pencil beam, or the like. By superimposing therapeutic radiation beams having a small irradiation field, it is possible to realize a complicated dose distribution and thus to concentrate radiation on an affected area.

### [Citation List]

### [Patent Literature]

[Patent Literature 1]
   Japanese Unexamined Patent Application, First Publication No. 2001-224698
[Patent Literature 2]
   Japanese Unexamined Patent Application, First Publication No. 2005-526578

### [Summary of Invention]

### [Technical Problem]

In intensity modulated radiation therapy, therapeutic radiation with an irradiation field smaller than an affected area is used as described above. Accordingly, even when an internal image based on the therapeutic radiation is acquired, the outline of an affected area cannot be specified and it is thus difficult to determine whether the affected area is accurately irradiated with the therapeutic radiation.

On the other hand, since therapeutic radiation having a small irradiation field is used in the intensity modulated radiation therapy, an irradiation dose per unit irradiation tends to increase in order to secure a dose for an affected area. Since a plurality of therapeutic radiation beams are superimposed, an irradiation time tends to extend. Accordingly, in intensity modulated radiation therapy, a risk of therapeutic radiation departing from an affected area is relatively high. For this reason, in intensity modulated radiation therapy, it is preferable to confirm that an affected area is accurately irradiated with therapeutic radiation.

Evidence for radiation therapy is also requested, and thus it is preferable to obtain records that an affected area is accurately irradiated with therapeutic radiation.

The present invention provides a therapy planning device, a planned therapy system, a therapy plan making method, and a program which can enhance convergence of therapeutic radiation, and through which it can be confirmed and recorded that an affected area is accurately irradiated with therapeutic radiation.

### [Solution to Problem]

According to an aspect of the present invention, a therapy planning device includes: a condition acquiring unit configured to acquire therapy conditions including the position of an affected area, a necessary dose for the affected area, and a threshold dose for an area other than the affected area; a first planning unit configured to plan a first irradiation of irradiating a first irradiation field including the entire affected area to radiation; and a second planning unit configured to make a therapy plan satisfying the therapy conditions in combination of the first irradiation planned by the first planning unit and irradiation of an irradiation field including only part of the affected area.

A therapy planning device according to another aspect of the present invention, in the above-described therapy planning device, the first planning unit sets a timing of the first irradiation based on a user's operation of designating at least one of an irradiation start timing, an irradiation end timing, and an intermediate timing between the irradiation start timing and the irradiation end timing.

A therapy planning device according to another aspect of the present invention, in the above-described therapy planning device, the first planning unit sets the timing of the first irradiation based on a user's operation of designating an acquisition frequency of a radiographic image through the first irradiation.

A therapy planning device according to another aspect of the present invention, in the above-described therapy planning device, the first planning unit sets the timing of the first irradiation based on a user's operation of designating an acquisition cycle of a radiographic image through the first irradiation.

A therapy planning device according to another aspect of the present invention, in the above-described therapy planning device, the first planning unit sets an irradiation dose of the first irradiation to a predetermined irradiation dose necessary for imaging.

A therapy planning device according to another aspect of the present invention, in the above-described therapy planning device, the first planning unit sets an irradiation dose of the first irradiation based on the necessary dose for the affected area in the therapy conditions.

A therapy planning device according to another aspect of the present invention, in the above-described therapy planning device, the first planning unit sets the first irradiation field by adding a predetermined margin to the affected area.

A therapy planning device according to another aspect of the present invention, the above-described therapy planning device further includes: a display unit configured to display an image of the affected area on a display screen; and an operation input unit configured to receive a user's operation of setting the first irradiation field, wherein the first planning unit sets the first irradiation field based on the user's operation of setting the first irradiation field received by the operation input unit.

According to another aspect of the present invention, a planned therapy system includes a therapy planning device; and a radiation therapy system, wherein the therapy planning device includes a condition acquiring unit configured to acquire therapy conditions including a position of an affected area, a necessary dose for the affected area, and a threshold dose for an area other than the affected area, a first planning unit configured to set a first irradiation field including the entire affected area, a timing of a first irradiation of irradiating the first irradiation field to radiation, and an irradiation dose in the first irradiation, a second planning unit configured to make a therapy plan satisfying the therapy conditions in combination of the first irradiation set by the first planning unit and irradiation of an irradiation field including only part of the affected area, and a therapy plan output unit configured to output the therapy plan made by the second planning unit to the radiation therapy system, and wherein the radiation therapy system includes a therapy plan acquiring unit configured to acquire the therapy plan output from the therapy plan output unit, an irradiation unit configured to emit radiation based on the therapy plan acquired by the therapy plan acquiring unit, and a radiographic image acquiring unit configured to acquire a radiographic image using radiation emitted by the irradiation unit in the first irradiation.

According to another aspect of the present invention, a therapy plan making method of a therapy planning device includes a condition acquiring step of acquiring therapy conditions including a position of an affected area, a necessary dose for the affected area, and a threshold dose for an area other than the affected area; a first planning step of planning a first irradiation of irradiating a first irradiation field including the entire affected area to radiation; and a second planning step of making a therapy plan satisfying the therapy conditions in combination of the first irradiation planned in the first planning step and irradiation of an irradiation field including only part of the affected area.

According to another aspect of the present invention, a program causes a computer serving as a therapy planning device to perform: a condition acquiring step of acquiring therapy conditions including a position of an affected area, a necessary dose for the affected area, and a threshold dose for an area other than the affected area; a first planning step of planning a first irradiation of irradiating a first irradiation field including the entire affected area to radiation; and a second planning step of making a therapy plan satisfying the therapy conditions in combination of the first irradiation planned in the first planning step and irradiation of an irradiation field including only part of the affected area.

### [Advantageous Effects of Invention]

According to the therapy planning device, the planned therapy system, the therapy plan making method, and the program, it is possible to enhance convergence of therapeutic radiation, and to confirm and record that an affected area is accurately irradiated with therapeutic radiation.

### [Brief Description of Drawings]

Fig. 1 is a schematic diagram illustrating a device configuration of a radiation therapy device according to an embodiment of the present invention.
Fig. 2 is a schematic block diagram illustrating a functional configuration of a planned therapy system according to the embodiment.
Fig. 3 is a diagram illustrating an example of an irradiation field in a first irradiation according to the embodiment.
Fig. 4 is a diagram illustrating an example of a radiographic image which is acquired in the first irradiation according to the first embodiment.
Fig. 5 is a diagram illustrating an example of an irradiation field in a second irradiation according to the embodiment.
Fig. 6 is a sequence diagram illustrating an example of a process flow when the planned therapy system according to the embodiment executes radiation therapy.
Fig. 7 is a diagram illustrating an example of a screen for setting a first irradiation field according to the embodiment.
Fig. 8 is a graph illustrating an example of a position of a leaf in a multi-leaf collimator according to the embodiment.

### [Description of Embodiments]

Hereinafter, embodiments of the present invention will be described with reference to the accompanying drawings.

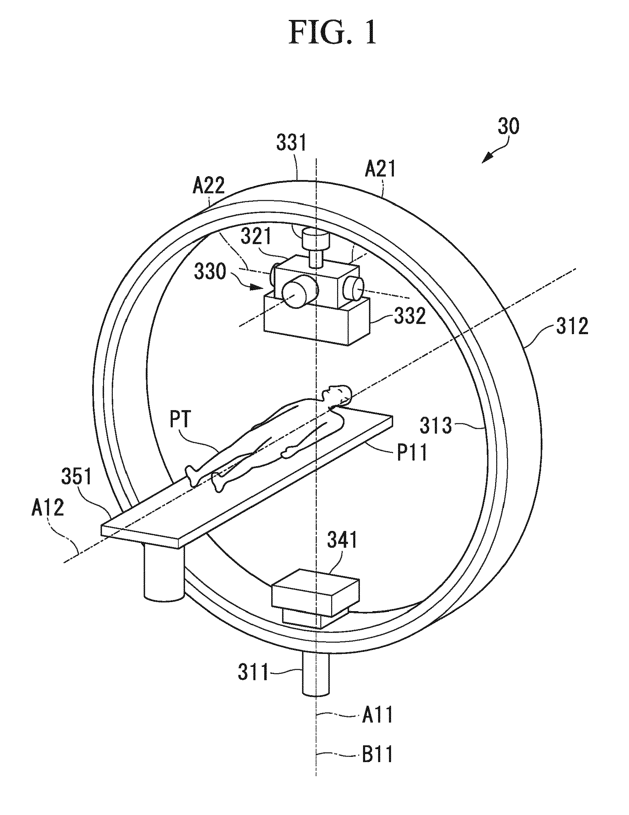

Fig. 1 is a schematic diagram illustrating a device configuration of a radiation therapy device according to an embodiment of the present invention. In Fig. 1, radiation therapy equipment 30 includes a gyration drive device 311, an O ring 312, a traveling gantry 313, a swinging mechanism 321, an irradiation unit 330, a sensor array 341, and a couch 351. The irradiation unit 330 includes a irradiation device 331 and a multi-leaf collimator 332.

The gyration drive device 311 supports the O ring 312 on a base to be rotatable about a rotation axis A11 and causes the O ring 312 to gyrate. The rotation axis A11 is an axis in the vertical direction.

The O ring 312 is formed in a ring shape centered on a rotation axis A12 and supports the traveling gantry 313 to be rotatable about the rotation axis A12. The rotation axis A12 is an axis in the horizontal direction (that is, an axis perpendicular to the vertical direction) and orthogonally intersects the rotation axis All at the isocenter P11. The rotation axis A12 is fixed with respect to the O ring 312. That is, the rotation axis A12 rotates about the rotation axis A11 with rotation of the O ring 312.

The O ring 312 rotates in order to rotate the traveling gantry 313 and the constituent units installed in the traveling gantry 313 about the rotation axis A11 together.

The traveling gantry 313 is formed in a ring shape centered on the rotation axis A12 and is disposed inside the O ring 312 to be concentric with the O ring 312. The radiation therapy equipment 30 includes a traveling drive device which is not illustrated and the traveling gantry 313 rotates about the rotation axis A12 with power from the traveling drive device.

The traveling gantry 313 rotates in order to rotate the constituent units installed in the traveling gantry 313 about the rotation axis A12 in a bundle.

The swinging mechanism 321 is fixed to the inside of the ring of the traveling gantry 313 and supports the irradiation unit 330 on the traveling gantry 313. The swinging mechanism 321 rotates the irradiation unit 330 about a pan axis A21 and also rotates the irradiation unit 330 about a tilt axis A22.

The pan axis A21 is an axis parallel to the rotation axis A12 and is fixed with respect to the traveling gantry 313. The swinging mechanism 321 causes the irradiation unit 330 to swing right and left with respect to the rotation axis A12 (accordingly, right and left with respect to a patient PT) by rotating the irradiation unit 330 about the pan axis A21.

The tilt axis A22 is an axis perpendicular to the pan axis A21 and is fixed with respect to the traveling gantry 313. The swinging mechanism 321 causes the irradiation unit 330 to swing in the direction of the rotation axis A12 (that is, up and down with respect to a patient PT) by rotating the irradiation unit 330 about the tilt axis A22.

The irradiation unit 330 is disposed inside the traveling gantry 313 and supported by the swinging mechanism 321 and emits therapeutic radiation B11.

The radiation irradiation device 331 emits therapeutic radiation B11. The irradiation device 331 is supported by the traveling gantry 313 with the swinging mechanism 321. Accordingly, after the irradiation device 331 is once directed to the isocenter P11 by adjustment of the swinging mechanism 321, the therapeutic radiation B11 substantially passes through the isocenter P11 even when the O ring 312 is rotated by the gyration drive device 311 or even when the traveling gantry 313 is rotated by the traveling drive device. Therefore, the irradiation device 331 rotates about the rotation axis A11 or the rotation axis A12 to emit the therapeutic radiation B11 to the isocenter P11 from various directions.

The multi-leaf collimator 332 adjusts the shape of an irradiation field when a patient is irradiated with the therapeutic radiation B11 by blocking part of the therapeutic radiation B11.

The sensor array 341 is disposed to face the irradiation device 331 at a position which the therapeutic radiation B11 from the irradiation device 331 reaches and is fixed to the inside of the ring of the traveling gantry 313.

The sensor array 341 is a device that is capable of detecting two-dimensional intensity of radiation, such as an EPID, receives the therapeutic radiation B11 passing through a patient PT and outputs an intensity signal. Here, "receiving" means receiving radiation.

The couch 351 is used for a patient PT to lie down on.

Hereinafter, the therapeutic radiation is simply referred to as "radiation" unless there is a particular need to be specific.

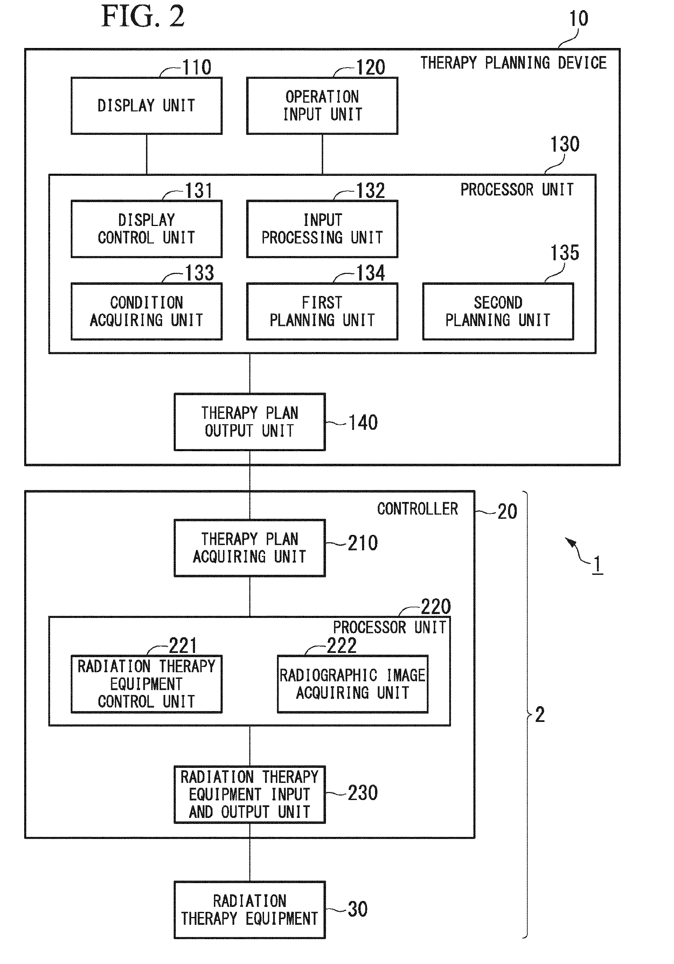

Fig. 2 is a schematic block diagram illustrating a functional configuration of a planned therapy system according to this embodiment. In Fig. 2, the planned therapy system 1 includes a therapy planning device 10 and a radiation therapy system 2. The therapy planning device 10 includes a display unit 110, an operation input unit 120, a processor unit 130, and a therapy plan output unit 140. The processor unit 130 includes a display control unit 131, an input processing unit 132, a condition acquiring unit 133, a first planning unit 134, and a second planning unit 135. The radiation therapy system 2 includes a controller 20 and radiation therapy equipment 30. The controller 20 includes a therapy plan acquiring unit 210, a processor unit 220, and a radiation therapy equipment input and output unit 230. The processor unit 220 includes a radiation therapy equipment control unit 221 and a radiographic image acquiring unit 222.

The radiation therapy system 2 executes radiation therapy based on a therapy plan made by the therapy planning device 10.

The controller 20 controls the constituent units of the radiation therapy equipment 30 to emit therapeutic radiation based on the therapy plan made by the therapy planning device 10. The controller 20 acquires a radiographic image resulting from the therapeutic radiation. The controller 20 may be configured of, for example, a computer or a dedicated circuit.

The therapy plan acquiring unit 210 acquires the therapy plan made and output by the therapy planning device 10.

The processor unit 220 controls the constituent units of the controller 20 to perform various processes. Particularly, the processor unit 220 causes the radiation therapy equipment 30 to execute radiation therapy based on the therapy plan, and acquires a radiographic image in the radiation therapy. The processor unit 220 is realized by causing a central processing unit (CPU) of the controller 20 to read a program from a storage device of the controller 20 and to execute the read program.

The radiation therapy equipment control unit 221 controls the constituent units of the radiation therapy equipment 30 based on the therapy plan acquired by the therapy plan acquiring unit 210. Particularly, the radiation therapy equipment control unit 221 causes the irradiation unit 330 (Fig. 1) to emit radiation based on the therapy plan acquired by the therapy plan acquiring unit 210.

The radiographic image acquiring unit 222 acquires a radiographic image based on the radiation which is emitted by the irradiation unit 330, which passes through an affected area or the like, and which is received by the sensor array 341. Particularly, the radiographic image acquiring unit 222 acquires a radiographic image based on the radiation emitted by the irradiation unit 330 in a first irradiation. As will be described later, the first irradiation is irradiation of an affected area as a whole, and the radiographic image acquiring unit 222 can acquire a radiographic image including the entire outline of the affected area by acquiring the radiographic image in the first irradiation. Accordingly, a person referring to the radiographic image can check whether the affected area is accurately irradiated with radiation.

The radiation therapy equipment input and output unit 230 serves as an interface for inputting and outputting a signal to and from the radiation therapy equipment 30.

The therapy planning device 10 makes a therapy plan for causing the radiation therapy system 2 to execute radiation therapy. The therapy planning device 10 may be configured of, for example, a computer or a dedicated circuit.

The display unit 110 includes a display screen such as a liquid crystal display or an organic electroluminescence (EL) display, and displays various images such as a moving image, a still image, or text (characters) on the display screen under the control of the display control unit 131. Particularly, the display unit 110 displays an image of an affected area, which is captured in advance, on the display screen when user's settings on irradiation such as an irradiation field are received. Here, the user is a person who executes radiation therapy using the planned therapy system 1, such as a doctor or a radiologist.

The operation input unit 120 includes an input device such as a touch sensor, which is disposed on the display screen of the display unit 110 to configure a touch panel, or a keyboard and receives a user's operation. Particularly, the operation input unit 120 receives a user's operation of setting a first irradiation field. Here, the first irradiation field is an irradiation field in the first irradiation and thus is set to an irradiation field including the entire affected area. For example, the operation input unit 120 receives a user's operation of setting the first irradiation field, which is a touching operation performed by a user in a state in which the display unit 110 displays an image of an affected area on the display screen.

The processor unit 130 controls the constituent units of the therapy planning device 10 to perform various processes. Particularly, the processor unit 130 makes a therapy plan for causing the radiation therapy system 2 to execute radiation therapy. The processor unit 130 is realized by causing a central processing unit (CPU) of the therapy planning device 10 to read a program from a storage device of the therapy planning device 10 and to execute the read program.

The display control unit 131 controls the display unit 110 to display various images.

The input processing unit 132 detects the user's operation received by the operation input unit 120. For example, the input processing unit 132 converts a signal output from the operation input unit 120 in response to a touch operation on the display screen into coordinates on the display screen.

The condition acquiring unit 133 acquires therapy conditions including information on a position of an affected area, a necessary dose for the affected area, and a threshold dose for an area other than the affected area. Here, the therapy conditions are conditions to be satisfied by the therapy plan made by the therapy planning device 10. For example, the condition acquiring unit 133 acquires the therapy conditions based on a user's operation which is received by the operation input unit 120, such as an input of a position of an affected area based on a touch operation and an input of a necessary dose and a threshold dose using a keyboard.

The first planning unit 134 plans a first irradiation. As described above, the first irradiation is an irradiation of a first irradiation field including the entire affected area to radiation.

The second planning unit 135 makes a therapy plan to satisfy the therapy conditions in combination of the first irradiation planned by the first planning unit 134 and irradiation of an irradiation field including only part of the affected area. Hereinafter, the irradiation of an irradiation field including only part of the affected area, which is included in the therapy plan by the second planning unit 135, is referred to as a "second irradiation."

The therapy plan output unit 140 outputs the therapy plan made by the second planning unit 135 to the controller 20.

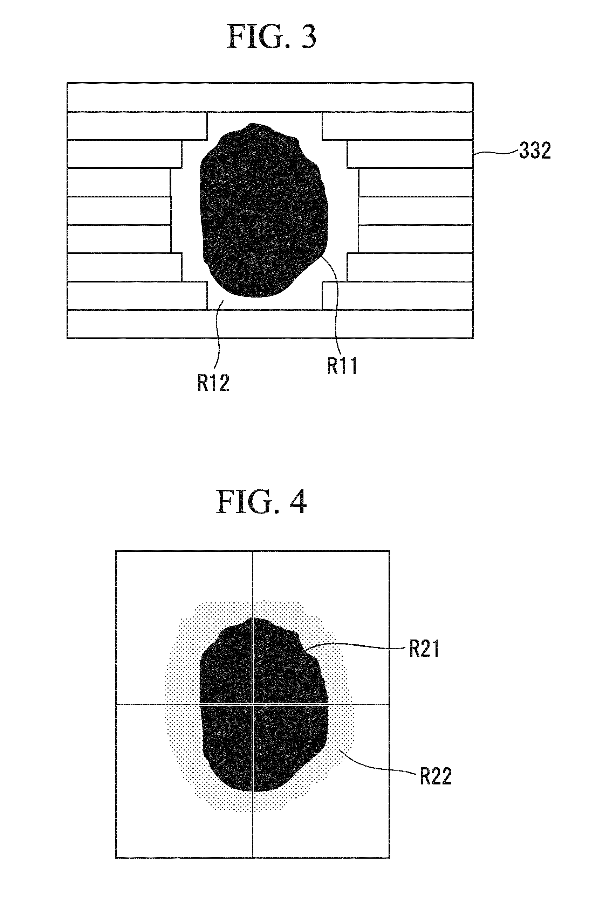

Now, the first irradiation and the second irradiation will be described with reference to Figs. 3 to 5.

Fig. 3 is a diagram illustrating an example of an irradiation field in the first irradiation. In the drawing, region R11 denotes a region of an affected area, and region R12 denotes an irradiation field (first irradiation field herein). As illustrated in Fig. 3, in the first irradiation, the multi-leaf collimator 332 adjusts an area of radiation such that the irradiation field includes the entire affected area.

Fig. 4 is a diagram illustrating an example of a radiographic image which is acquired in the first irradiation. In the drawing, region R21 denotes a region of an affected area in the radiographic image, and region R22 denotes an irradiation field in the radiographic image.

Here, the affected area is an area which is actively irradiated with radiation and may be one of a clinical target volume (CTV), an internal target volume (ITV), and a planning target volume (PTV).

For example, a planning target volume may be set as an affected area, and a region obtained by adding a predetermined margin to the entire planning target volume may be set as the irradiation field in the first irradiation. In this case, it can be confirmed that the entire planning target volume is included in the irradiation field from an image of a gross tumor volume (GTV) or an image of another organ and thus it can be confirmed that the affected area is accurately irradiated with radiation.

Alternatively, a clinical target volume may be used as an affected area and a region obtained by adding a predetermined margin to the entire clinical target volume may be set as the irradiation field in the first irradiation. In this case, similarly to the case of the planning target volume, it can be confirmed that the entire clinical target volume is included in the irradiation field and thus it can be confirmed that the affected area is accurately irradiated with radiation.

Similarly, when an internal target volume is used as an affected area, it can be confirmed that the affected area is accurately irradiated with radiation.

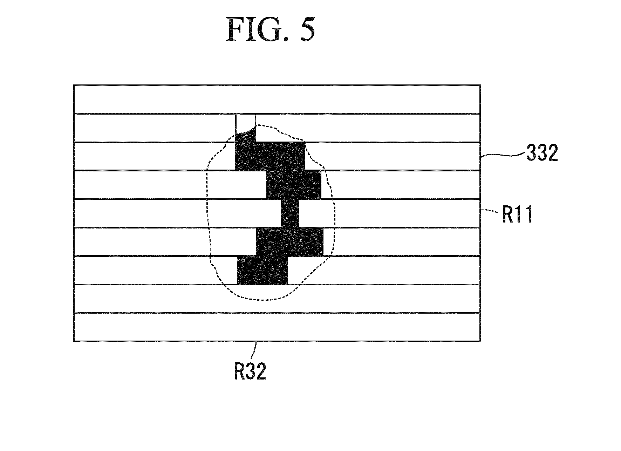

Fig. 5 is a diagram illustrating an example of an irradiation field in the second irradiation. In Fig. 5, region R11 denotes a region of an affected area similarly to the case of Fig. 3. Region R32 denotes an irradiation field. As illustrated in Fig. 5, in the second irradiation, the multi-leaf collimator 332 narrows radiation such that the irradiation field includes only part of the affected area.

In this way, by causing the multi-leaf collimator 332 to narrow the radiation to form an irradiation field including only part of the affected area, it is possible to enhance convergence of radiation as in the case of intensity modulated radiation therapy.

For example, in a state in which an affected area is located at the isocenter P11 (Fig. 1), the traveling drive device rotates the O ring 312, and the irradiation unit 330 emits radiation to the affected area from different directions while turning around the affected area with the rotation of the O ring 312. At this time, in order to narrow a shape of an irradiation area to be irradiated with radiation to correspond to the shape of the affected area and to exclude an area overlapping the affected area and having a low allowable dose from the irradiation field, the multi-leaf collimator 332 narrows the shape of the irradiation area such that the irradiation field includes only part of the affected area. Accordingly, it is possible to concentrate radiation on the affected area and to reduce an irradiation dose for neighboring normal areas.

Here, when the irradiation field includes only part of the affected area (that is, when the second irradiation is performed), a person referring to a radiographic image is considered unable to identify the outline of the affected area using the radiographic image which is acquired by the radiographic image acquiring unit 222 from radiation received by the sensor array 341. Since the outline of the affected area cannot be identified, it is difficult to determine whether the affected area is accurately irradiated with radiation.

Therefore, by causing the first planning unit 134 to set the first irradiation, the radiographic image acquiring unit 222 acquires a radiographic image including the entire affected area as in the example illustrated in Fig. 4. Accordingly, a person referring to the radiographic image can confirm that the irradiation field includes the entire affected area and thus can confirm that the affected area is accurately irradiated with radiation.

Here, even when the radiation therapy system 2 performs the first irradiation for imaging independently of the irradiation for therapy, a person referring to the acquired radiographic image can confirm that the irradiation field includes the entire affected area and thus can confirm that the affected area is accurately irradiated with therapeutic radiation. However, in this case, the irradiation dose increases and the irradiation dose for normal areas also increases, in comparison with the case in which the irradiation for imaging is not performed.

Therefore, the second planning unit 135 makes a therapy plan in which the irradiation dose of the first irradiation is included in the irradiation dose for therapy. Accordingly, it is possible to suppress an increase in the irradiation dose for a normal area and to acquire a radiographic image including the entire affected area.

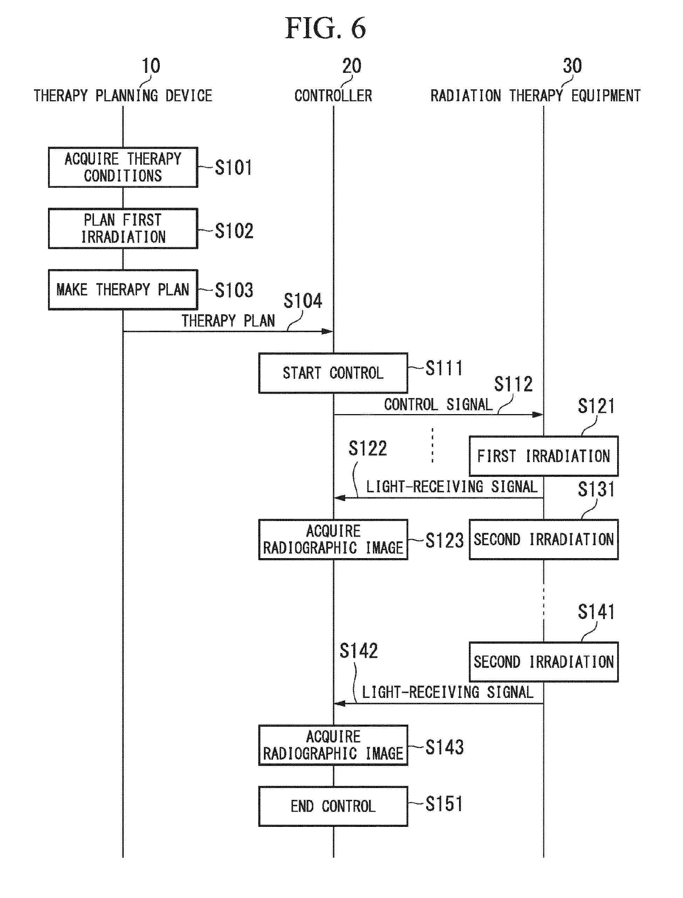

The operation of the planned therapy system 1 will be described below with reference to Fig. 6.

Fig. 6 is a sequence diagram illustrating an example of a process flow when the planned therapy system 1 executes radiation therapy. The planned therapy system 1 starts the process flow illustrated in Fig. 6, for example, when a user's operation of instructing to make a therapy plan is received.

In the process flow illustrated in Fig. 6, first, the condition acquiring unit 133 acquires therapy conditions (sequence S101). For example, as described above, the condition acquiring unit 133 acquires the therapy conditions based on a user's operation which is received by the operation input unit 120, such as an input of a position of an affected area based on a touch operation and an input of a necessary dose and a threshold dose using a keyboard.

Then, the first planning unit 134 makes a plan of the first irradiation (sequence S102). For example, the first planning unit 134 makes a plan including setting of a timing of the first irradiation, the first irradiation field, and the irradiation dose in the first irradiation. Single radiation therapy may include a single first irradiation or may include a plurality of first irradiations. When a plurality of first irradiations is included, the first planning unit 134 makes a plan of the first irradiation for each first irradiation.

Here, the first planning unit 134 can use various methods as a method of setting the timing of a first irradiation.

For example, the first planning unit 134 may set the timing of a first irradiation to a timing selected in response to a user's operation of designating (selecting) one or more of an irradiation start timing, an irradiation end timing, and an intermediate timing between the irradiation start timing and the irradiation end timing. Here, the irradiation start timing means a timing at which the irradiation is started for each port. The irradiation end timing means a timing at which the irradiation in the corresponding port is ended.

The first planning unit 134 may receive designation of a user of the intermediate timing between the irradiation start timing and the irradiation end timing as designation of a temporal position in the entire time from the irradiation start timing to the irradiation end timing. Alternatively, when the radiation therapy equipment 30 performs the irradiation while rotating the ring, the first planning unit 134 may receive the designation of a user of the intermediate timing between the irradiation start timing and the irradiation end timing as a designation of a rotation angle of the O ring 312.

Alternatively, the operation input unit 120 may receive a user's operation of designating the acquisition frequency of a radiographic image through the first irradiation and the first planning unit 134 may set the timing of the first irradiation based on the user's operation. For example, the first planning unit 134 divides the entire time from the irradiation start timing to the irradiation end timing based on the frequency designated by the user and sets the acquired timings as the timing of the first irradiation. At this time, the irradiation start timing, the irradiation end timing, or both thereof may be included or may not be included in the timing of the first irradiation.

Alternatively, the operation input unit 120 may receive a user's operation of designating an acquisition cycle of a radiographic image through the first irradiation, and the first planning unit 134 may set the timing of the first irradiation based on the user's operation. For example, the first planning unit 134 sets the timing of the first irradiation at the irradiation start timing and for each designated cycle.

The first planning unit 134 can use various methods as the method of setting the irradiation dose in the first irradiation.

For example, the first planning unit 134 may set a predetermined irradiation dose necessary for imaging as the irradiation dose in the first irradiation. Specifically, the first planning unit 134 stores radiation intensity (irradiation dose per unit time) suitable for acquiring an internal image as a radiographic image depending on performance of the sensor array 341 in advance, and sets the radiation intensity as the radiation intensity in the first irradiation.

Alternatively, the first planning unit 134 may set the irradiation dose in the first irradiation based on the necessary dose for an affected area in the therapy conditions. For example, the first planning unit 134 calculates 20% of a dosage, which is set based on the necessary dose, as the irradiation dose in the entire first irradiation and sets a quotient obtained by dividing the calculated irradiation dose by the number of first irradiations (irradiation frequency) as the irradiation dose in the single first irradiation. The first planning unit 134 adjusts the radiation intensity to radiation intensity suitable for acquiring an internal image as a radiographic image by adjusting an irradiation time in the single first irradiation.

The first planning unit 134 can use various methods as the method of setting the first irradiation field.

For example, the first planning unit 134 may set the first irradiation field by adding a predetermined margin to a region of an affected area. As described above, the affected area may be one of a clinical target volume, an internal target volume, and a planning target volume.

Alternatively, the first planning unit 134 may set the first irradiation field based on a user's operation of setting the first irradiation field, which is received by the operation input unit 120.

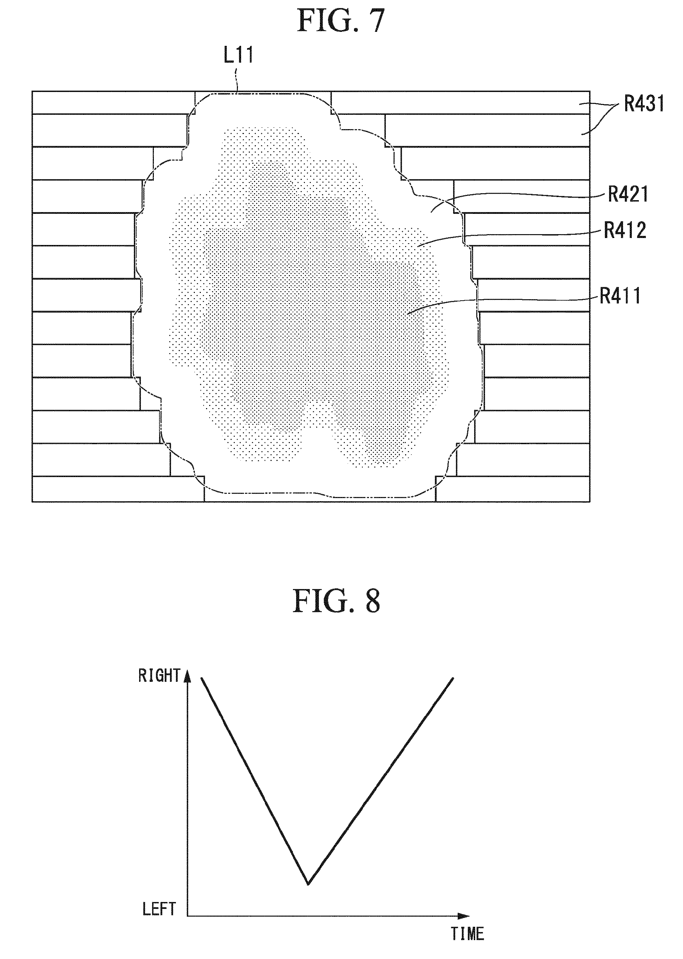

Fig. 7 is a diagram illustrating an example of a screen for setting the first irradiation field. In the example illustrated in Fig. 7, the display unit 110 displays an image of an affected area, in which region R411 denotes a gross tumor volume. Region R412 denotes a clinical target volume.

For example, a user determines a planning target volume, which is obtained by adding an internal margin (IM) for movement of internal organs or the like and a setup margin (SM) in irradiation to region R412 of the clinical target volume, as the first irradiation field. Then, the user designates the first irradiation field by touching the display screen based on the determined first irradiation field. In the example illustrated in Fig. 7, line L11 denotes a locus touched by the user and region L421 inside line L11 denotes the designated irradiation field. Region R431 denotes narrowing of radiation which is performed by the multi-leaf collimator 332 based on the designated irradiation field.

Referring to Fig. 6 again, after sequence S102, the second planning unit 135 makes a therapy plan (sequence S103). As described above, the second planning unit 135 makes a therapy plan satisfying the therapy conditions in combination of the first irradiation (all of the first irradiations when there are a plurality of first irradiations) and a second irradiation.

Here, the second planning unit 135 may make a therapy plan with a decrease in a moving distance of each leaf of the multi-leaf collimator 332 as one objective.

Fig. 8 is a graph illustrating an example of a position of a leaf in the multi-leaf collimator 332. In Fig. 8, the horizontal axis denotes time. The vertical axis denotes a position of a leaf, in which the upper side denotes a position closer to the right and the lower side denotes a position closer to the left. The leaf illustrated in Fig. 8 is disposed on the right side of the multi-leaf collimator 332 and is located at a position close to the right in a shape in which an aperture of the multi-leaf collimator 332 is large.

In the example illustrated in Fig. 8, the first planning unit 134 sets the first irradiation at a radiation therapy start timing and a radiation therapy end timing and the second planning unit 135 makes a therapy plan such that the leaf is located at a position closest to the right at the radiation therapy start timing and the radiation therapy end timing. Here, the radiation therapy start timing means a timing at which the radiation therapy equipment 30 starts an operation based on the therapy plan under the control of the controller 20. The radiation therapy end timing is a timing at which the radiation therapy equipment 30 ends (completes) the operation based on the therapy plan under the control of the controller 20.

On the other hand, in the time between the radiation therapy start timing and the radiation therapy end timing, the second planning unit 135 reduces an aperture portion of the multi-leaf collimator 332 and causes the radiation therapy system 2 to perform the second irradiation. At this time, the multi-leaf collimator 332 gradually moves from a position close to the right to a position close to the left and then gradually moves to a position close to the right such that the graph illustrated in Fig. 8 monotonically increases after monotonically decreasing, whereby it is possible to reduce the moving distance of the leaf.

Accordingly, since the time for waiting for movement of a leaf can be shortened, it is possible to shorten the therapy time. When the multi-leaf collimator 332 is activated to continuously perform irradiation, it is possible to prevent the irradiation from stopping due to discontinuity of a leaf position.

Referring to Fig. 6 again, after sequence S103, the therapy plan output unit 140 outputs the therapy plan made by the second planning unit 135 to the controller 20 (sequence S104).

In the controller 20, when the therapy plan acquiring unit 210 acquires the therapy plan and a user's operation of instructing to start the radiation therapy is input, the radiation therapy equipment control unit 221 starts control of the radiation therapy equipment 30 (sequence S111).

The radiation therapy equipment control unit 221 having started the control of the radiation therapy equipment 30 controls the radiation therapy equipment 30 by outputting a control signal to the radiation therapy equipment 30 via the radiation therapy equipment input and output unit 230 based on the therapy plan (sequence S 112). Thereafter, the radiation therapy equipment control unit 221 outputs the control signal to the radiation therapy equipment 30 via the radiation therapy equipment input and output unit 230 continuously or periodically until the radiation therapy ends.

The radiation therapy equipment 30 having received the control signal from the radiation therapy equipment control unit 221 operates in accordance with the control signal and performs the first irradiation or the second irradiation (sequences S121, S131, and S141).

Then, the radiation therapy equipment 30 outputs a light-receiving signal indicating a two-dimensional intensity of radiation detected by the sensor array 341 to the controller 20 at least at the timing of performing the first irradiation (sequences S122 and S 142).

In the controller 20 having acquired the light-receiving signal from the radiation therapy equipment 30, the radiographic image acquiring unit 222 acquires a radiographic image by converting the light-receiving signal into radiographic image data (sequences S123 and S143).

The radiation therapy equipment 30 may output the light-receiving signal at only the timing of performing the first irradiation, may continuously output the light-receiving signal, or may output the light-receiving signal at only the timing of performing the second irradiation. Accordingly, the radiographic image acquiring unit 222 may acquire only the radiographic image through the first irradiation or may additionally acquire the radiographic image through the second irradiation.

When the therapy plan made by the second planning unit 135 is completed, the radiation therapy equipment control unit 221 ends the control of the radiation therapy equipment 30 (sequence S151).

Thereafter, the process flow illustrated in Fig. 6 ends.

As described above, the first planning unit 134 plans the first irradiation of irradiating the first irradiation field including the entire affected area to radiation. Then, the second planning unit 135 makes a therapy plan satisfying the therapy conditions in combination of the first irradiation planned by the first planning unit 134 and the second irradiation which is irradiation of an irradiation field including only part of the affected area.

Accordingly, it is possible to enhance convergence of radiation and to confirm and record that the affected area is accurately irradiated with radiation. That is, since the second planning unit 135 makes a therapy plan including the second irradiation, it is possible to enhance convergence of radiation similarly to the case of intensity modulated radiation therapy. Since the first planning unit 134 plans the first irradiation and the second planning unit 135 makes a therapy plan including the first irradiation, it is possible to acquire a radiographic image including the entire affected area. Accordingly, a person referring to the radiographic image can confirm that the irradiation field includes the entire affected area and thus can confirm that the affected area is accurately irradiated with radiation.

The first planning unit 134 sets the timing of the first irradiation based on a user's operation of designating at least one of an irradiation start timing, an irradiation end timing, and an intermediate timing between the irradiation start timing and the irradiation end timing.

Accordingly, the user can designate the timing of the first irradiation with a simple operation of designating (selecting) at least one of the irradiation start timing, the irradiation end timing, and an intermediate timing between the irradiation start timing and the irradiation end timing.

When it can be confirmed that an affected area is accurately irradiated with radiation at the irradiation start timing and the irradiation end timing in each port, it can be assumed that the affected area is accurately irradiated with radiation through all the ports.

Even when the irradiation time is long, the possibility of the affected area being accurately irradiated with radiation through all the ports becomes much higher by confirming that an affected area is accurately irradiated with radiation at an intermediate timing between the irradiation start timing and the irradiation end timing.

The first planning unit 134 sets the timing of the first irradiation based on the user's operation of designating the acquisition frequency of a radiographic image through the first irradiation.

Accordingly, the user can cause the first planning unit 134 to set the timing of the first irradiation depending on a desired frequency with a simple operation of designating the acquisition frequency of a radiographic image through the first irradiation.

The first planning unit 134 sets the timing of the first irradiation based on the user's operation of designating the acquisition cycle of a radiographic image through the first irradiation.

Accordingly, the user can cause the first planning unit 134 to set the timing of the first irradiation depending on a desired cycle with a simple operation of designating the acquisition cycle of a radiographic image through the first irradiation.

The first planning unit 134 sets a predetermined irradiation dose necessary for imaging as the irradiation dose in the first irradiation. Specifically, as described above, the first planning unit 134 sets the radiation intensity in the first irradiation to a radiation intensity suitable for acquiring an internal image as a radiographic image depending on performance of the sensor array 341.

Accordingly, the radiographic image acquiring unit 222 can acquire a vivid internal image as a radiographic image in the first irradiation.

The first planning unit 134 sets an irradiation dose in the first irradiation based on the necessary dose for an affected area in the therapy conditions.

Accordingly, the second planning unit 135 can be expected to be able to easily make a therapy plan satisfying the therapy conditions. For example, when the first planning unit 134 sets the irradiation dose in the entire first irradiation to 20% of a dosage which is set based on the necessary dose, the second planning unit 135 can make a therapy plan in which 80% of the dosage is set as the irradiation dose in the second irradiation.

Therefore, it is possible to secure a degree of freedom when the second planning unit 135 makes a therapy plan.

The first planning unit 134 sets the first irradiation field by adding a predetermined margin to a region of an affected area.

Accordingly, it is possible to reduce a burden on a user of designating the first irradiation field. That is, a user need only designate a region of an affected area and need not separately designate the first irradiation field.

The display unit 110 displays an image of an affected area on the display screen, the operation input unit 120 receives a user's operation of setting the first irradiation field, and the first planning unit 134 sets the first irradiation field based on the user's operation of setting the first irradiation field which is received by the operation input unit 120.

Accordingly, the user can designate an appropriate first irradiation field with reference to the image of the affected area.

The processes of the constituents may be performed by recording a program for realizing all or some functions of the therapy planning device 10 on a computer-readable recording medium and causing a computer system to read and execute the program recorded on the recording medium. Here, the "computer system" includes an OS or hardware such as peripherals.

Here, the "computer system" may include a homepage providing environment (or display environment) when a WWW system is used.

Examples of the "computer-readable recording medium" include a portable medium such as a flexible disk, a magneto-optical disc, a ROM, and a CD-ROM and a storage device such as a hard disk built in a computer system. The "computer-readable recording medium" may include a medium that temporarily holds a program for a predetermined time, like a volatile memory (RAM) in a computer system serving as a server or a client when a program is transmitted via a network such as the Internet or a communication circuit such as a telephone circuit. The program may be configured to realize some of the above-mentioned functions or may be configured to realize the above-mentioned functions in combination with a program recorded in advance in a computer system.

While the embodiments of the present invention have been described in detail with reference to the accompanying drawings, the specific configuration is not limited to the embodiments and includes a design or the like without departing from the gist of the present invention.

### [Industrial Applicability]

According to the therapy planning device, the planned therapy system, the therapy plan making method, and the program, it is possible to enhance convergence of therapeutic radiation and to confirm and record that an affected area is accurately irradiated with therapeutic radiation.

### [Reference Signs List]

1 Planned therapy system
10 Therapy planning device
110 Display unit
120 Operation input unit
130 Processor unit
131 Display control unit
132 Input processing unit
133 Condition acquiring unit
134 First planning unit
135 Second planning unit
140 Therapy plan output unit
2 Radiation therapy system
20 Controller
210 Therapy plan acquiring unit
220 Processor unit
221 Radiation therapy equipment control unit
222 Radiographic image acquiring unit
230 Radiation therapy equipment input and output unit
30 Radiation therapy device

## Claims

1. A therapy planning device comprising:
a condition acquiring unit configured to acquire therapy conditions including a position of an affected area, a necessary dose for the affected area, and a threshold dose for an area other than the affected area;
a first planning unit configured to plan a first irradiation of irradiating a first irradiation field including the entire affected area to radiation; and
a second planning unit configured to make a therapy plan satisfying the therapy conditions in combination of the first irradiation planned by the first planning unit and irradiation of an irradiation field including only part of the affected area.

2. The therapy planning device according to claim 1, wherein the first planning unit sets a timing of the first irradiation based on a user's operation of designating at least one of an irradiation start timing, an irradiation end timing, and an intermediate timing between the irradiation start timing and the irradiation end timing.

3. The therapy planning device according to claim 1 or 2, wherein the first planning unit sets the timing of the first irradiation based on a user's operation of designating an acquisition frequency of a radiographic image through the first irradiation.

4. The therapy planning device according to claim 1 or 2, wherein the first planning unit sets the timing of the first irradiation based on a user's operation of designating an acquisition cycle of a radiographic image through the first irradiation.

5. The therapy planning device according to any one of claims 1 to 4, wherein the first planning unit sets an irradiation dose of the first irradiation to a predetermined irradiation dose necessary for imaging.

6. The therapy planning device according to any one of claims 1 to 4, wherein the first planning unit sets an irradiation dose of the first irradiation based on the necessary dose for the affected area in the therapy conditions.

7. The therapy planning device according to any one of claims 1 to 6, wherein the first planning unit sets the first irradiation field by adding a predetermined margin to the affected area.

8. The therapy planning device according to any one of claims 1 to 6, further comprising:
a display unit configured to display an image of the affected area on a display screen; and
an operation input unit configured to receive a user's operation of setting the first irradiation field,
wherein the first planning unit sets the first irradiation field based on the user's operation of setting the first irradiation field received by the operation input unit.

9. A planned therapy system comprising:
a therapy planning device; and
a radiation therapy system,
wherein the therapy planning device includes:
a condition acquiring unit configured to acquire therapy conditions including a position of an affected area, a necessary dose for the affected area, and a threshold dose for an area other than the affected area,
a first planning unit configured to set a first irradiation field including the entire affected area, a timing of a first irradiation of irradiating the first irradiation field to radiation, and an irradiation dose in the first irradiation,
a second planning unit configured to make a therapy plan satisfying the therapy conditions in combination of the first irradiation set by the first planning unit and irradiation of an irradiation field including only part of the affected area, and
a therapy plan output unit configured to output the therapy plan made by the second planning unit to the radiation therapy system, and
wherein the radiation therapy system includes:
a therapy plan acquiring unit configured to acquire the therapy plan output from the therapy plan output unit,
an irradiation unit configured to emit radiation based on the therapy plan acquired by the therapy plan acquiring unit, and
a radiographic image acquiring unit configured to acquire a radiographic image using radiation emitted by the irradiation unit in the first irradiation.

10. A therapy plan making method of a therapy planning device, comprising:
a condition acquiring step of acquiring therapy conditions including a position of an affected area, a necessary dose for the affected area, and a threshold dose for an area other than the affected area;
a first planning step of planning a first irradiation of irradiating a first irradiation field including the entire affected area to radiation; and
a second planning step of making a therapy plan satisfying the therapy conditions in combination of the first irradiation planned in the first planning step and irradiation of an irradiation field including only part of the affected area.

11. I A program causing a computer serving as a therapy planning device to perform:
a condition acquiring step of acquiring therapy conditions including a position of an affected area, a necessary dose for the affected area, and a threshold dose for an area other than the affected area;
a first planning step of planning a first irradiation of irradiating a first irradiation field including the entire affected area to radiation; and
a second planning step of making a therapy plan satisfying the therapy conditions in combination of the first irradiation planned in the first planning step and irradiation of an irradiation field including only part of the affected area.
